(19) Europäisches Patentamt
European Patent Office
Office européen des brevets

(11) Publication number: **0 008 657**
**B1**

(12) **EUROPEAN PATENT SPECIFICATION**

(45) Date of publication of patent specification: **30.03.83**

(21) Application number: **79102614.9**

(22) Date of filing: **24.07.79**

(51) Int. Cl.³: **C 07 C 101/72,**
C 07 C 101/28,
C 07 D 209/20,
C 07 D 233/64,
A 61 K 31/195,
A 61 K 31/405,
A 61 K 31/415

(54) **Alpha-vinyl-alpha-aminoacids and their esters and pharmaceutical compositions containing them.**

(30) Priority: **24.07.78 US 927440**

(43) Date of publication of application:
**19.03.80 Bulletin 80/6**

(45) Publication of the grant of the patent:
**30.03.83 Bulletin 83/13**

(84) Designated Contracting States:
**AT BE CH DE FR GB IT LU NL SE**

(56) References cited:
**EP - A - 0 000 035**
**DE - A - 2 827 805**
**US - A - 4 103 089**

**TETRAHEDRON LETTERS No. 32 (1977)**
**TETRAHEDRON LETTERS No. 41 (1977).**
**BIOCHEMISTRY, 15 (1976).**

(73) Proprietor: **MERCK & CO. INC.**
**126, East Lincoln Avenue P.O. Box 2000**
**Rahway New Jersey 07065 (US)**

(72) Inventor: **Patchett, Arthur Allan**
**1090 Minisink Way**
**Westfield, N.J. 07090 (US)**
Inventor: **Taub, David**
**54 Wistar Avenue**
**Metuchen, N.J. 08840 (US)**

(74) Representative: **Abitz, Walter, Dr.-Ing. et al,**
**Abitz, Morf, Gritschneder P.O. Box 86 01 09**
**D-8000 München 86 (DE)**

**EP 0 008 657 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Alpha-vinyl-alpha-aminoacids and their esters and pharmaceutical compositions containing them

## Background of the invention

The present invention is concerned with novel α-vinyl-α-amino acids having pharmaceutical utility.

α-Vinyl-3,4-dihydroxyphenylalanine is described in Tetrahedron Letters No. 32, p. 2745—2748 (1977) and No. 41, p. 3689—3692 (1977). Vinylglycine and α-methyl-α-amino vinylacetic acid are also known compounds [Acta. Chem. Scand., Sec. B *28* 317—321 (1974); Biochemistry *15* 3070—76 (1976); Japanese Patent Application 70, 15011].

Certain novel α-vinyl amino acids have been discovered. These compounds have pharmaceutical activity.

## Summary of the invention

The invention is concerned with α-vinyl-α-aminopropionic acids and esters of the following formula:

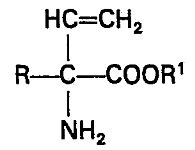

and pharmaceutically acceptable salts thereof wherein R is

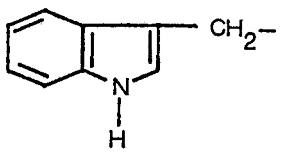 ,

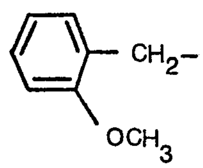 ,

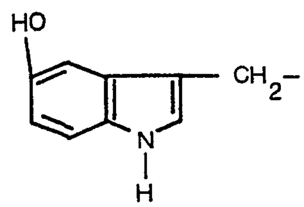 ,

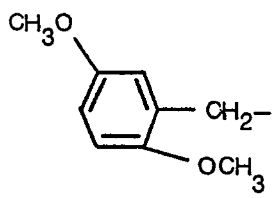 ,

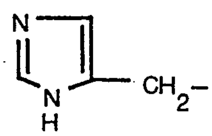 ,

or

$H_2N—(CH_2)_2—CH_2—$

and $R^1$ is H or $C_1—C_{18}$ alkyl.

The pharmaceutically acceptable salts are the acid addition salts of the Formula I compounds. Suitable acids include organic as well as inorganic acids. Preferred salts are the hydrohalides such as the hydrochlorides, hydrobromides and hydroiodides. Most preferred salts are the hydrochlorides.

The compounds of Formula I have a chiral center and this occurs in optically active forms, i.e., optical isomers. These isomers are conventionally designated as D and L, d and l, (+) and (—), (S) and (R) or by a combination of these symbols. Where a compound name or formula does not indicate a specific isomer, all forms are included, i.e., the individual isomers, mixtures thereof and racemates of the optical isomers, the L-form is preferred.

$R^1$ may be H or a $C_1—C_{18}$alkyl group. Examples of suitable alkyl groups are octadecyl, 2-ethylhexyl, lauryl, hexyl, heptyl, isopropyl, methyl and the like. Preferred $R^1$ groups are H and $C_1—C_6$ hydrocarbon alkyl. Most preferred $R^1$ groups are H and ethyl.

One class of preferred R group is the imidazolyl moiety:

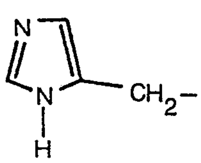

Illustrative examples of useful compounds are:

2-vinyl-2-amino-3-(2-methoxyphenyl)propionic acid,

2-vinyl-2-amino-3-(2,5-dimethoxy-phenyl)propionic acid,
2-vinyl-2-amino-3-(2-imidazolyl)propionic acid,
2-vinyl-2-amino-5-aminopentanoic acid,
2-vinyl-2-amino-3-(2-methoxyphenyl)propionic acid ethyl ester, and the like.

The compounds of the present invention have pharmaceutical activity and exhibit enzyme inhibition[(1)]. Compounds of Formula I where R is said methoxy or hydroxy phenyl substituents have anti-hypertensive activity. Thus, they are useful for treating hypertension (high blood pressure) in human beings alone or combined with carbidopa.

For treating hypertension, these compounds may be administered to the hypertensive patient orally or parenterally. Suitable conventional dosage forms are used such as tablets, troches, capsules, liquid formulations, e.g., solutions, dispersions, emulsions and the like. These pharmaceutical compositions may contain conventional pharmaceutically acceptable compounding ingredients, i.e., diluents, carriers, etc. and conventional preparative procedures are used.

The daily antihypertensive dosage may be varied as required. In general, a daily dosage range for the hypertensive patient is about 50 mg to about 5000 mg, preferably from about 100 to about 3500 mg and more preferably from about 250 mg to about 1500 mg.

Compounds of Formula I where R is the imidazolyl moiety are pharmaceutically useful as gastric secretion inhibitors or as antihistamine antiallergics. Effective daily dosages for these compounds may be varied as necessary. Daily dosage may range, e.g., from about 100 mg to about 3000 mg. Dosage forms suitable for the administration made, e.g., oral, insufflation, parenteral are used.

Compounds of Formula I where R is $NH_2—CH_2—CH_2—CH_2—$ are useful as antitumor agents. Mode of administration of these compounds may be oral or parenteral. Effective daily dosage may be varied and may range from about 100 mg to about 5000 mg. Suitable dosage forms prepared using conventional procedures and compounding ingredients i.e., diluents, carriers etc., are used.

The present compounds may be prepared by appropriate reduction of $\alpha$-ethynyl intermediates of the formula:

$$R—\underset{NH_2}{\overset{C\equiv CH}{C}}—COOR^1$$

This reduction process is described in the Tetrahedron Letters references identified above.

Another process for preparing compounds of Formula I is by the reaction illustrated by the following equations:

Reaction sequence A

$$CH_3—CH=\underset{N=CH—Ph}{C}—COO—CH_3$$

(a)

↓STRONG BASE

↓alkylating agent

$$CH_2=CH—\underset{NH_2}{\overset{R}{C}}—COOCH_3$$

(b)

↓hydrolysis

$$CH_2=CH—\underset{NH_2}{\overset{R}{C}}—COOH$$

Where R is containing a hydroxy group, this group should be protected, e.g., as phenyl—$CH_2$—O—. In this instance, this protective group in compound (b) is removed before hydrolysis.

The following examples illustrate preparation of representative compounds of Formula I using reaction sequence (A). All temperatures are in degrees Celsius.

---

[(1)]This attribute makes them useful as biochemical reagents.

Example I

(A) N-benzylidene-2-aminocrotonic acid methyl ester

To a well-stirred slurry of 16.6 g of (DL)-β-chloro-α-amino-butyric acid methyl ester hydrochloride in 160 ml of methylene chloride was added at 5°C, 9.0 ml of benzaldehyde, 7.5 g of anhydrous magnesium sulfate and 12.3 ml of triethylamine. After 20 hours the reaction mixture was diluted with 200 ml of water and the layers were separated. After three extractions of the aqueous layer with 100 ml of methylene chloride, the combined organic portions were washed with brine and dried with 5 g of anhydrous magnesium sulfate, giving after evaporation of solvent 20.8 g of crude (DL)-N-benzylidine-β-chloro-α-amino-crotonic acid methyl ester. This was dissolved in 160 ml of methylene chloride, and 13.0 ml of 1,5-dizabicyclo[5.4.0]undec-5-ene was added with stirring to the cooled (5°C) solution. After 90 minutes the reaction mixture was washed with 100 ml of water, then dried with 10 g of anhydrous sodium sulfate. The residue after evaporation of solvent was filtered through neutral alumina (70 g) in 10:1 hexane:ether, providing 14.5 g of pure N-benzylidine-2-aminocrotonic acid methyl ester as a 60:40 mixture of E:Z isomers. NMR ($CDCl_3$) showed: E isomer; 2.00 (3H, d, J=7), 3.77 (3H, s), 5.88 (1H, q, J=7), 8.18 (1H, s), 7.1—7.8 (5H, m); Z isomer; 1.83 (3H, d, J=7), 3.73 (3H, s), 6.50 (1H, q, J=7), 8.40 (1H, s), 7.1—7.8 (5H, m). TLC showed overlapping spots, $R_f$=0.3 (silica gel, 5:1 hexane:ether). IR ($CHCl_3$) showed 1720, 1660, 1575 $cm^{-1}$. Mass Spectrum, Calcd. for $C_{12}H_{13}NO_2$, 203.0946 Found: 203.0946.

(B) Benzyloxy-α-vinyltyrosine methyl ester

A solution of lithium hexamethyldisilazide was prepared by adding 5.5 mmol of butyllithium (2.2 M solution in hexane) to a cooled (—70°C) solution of 1.15 ml of hexamethyldisilazane in 12.5 ml of tetrahydrofuran. To this solution was added first 3.5 ml of hexamethylphosphoramide and then a solution of 1.15 g of N-benzylidene-2-aminocrotonic acid methyl ester in 12.5 ml of tetrahydrofuran. After 45 minutes, a solution of N-benzylidine-3-amino-1-bromo-propane in 7.5 ml of tetrahydrofuran was added. Stirring was continued for one hour at —70°C and then for two hours at room temperature. The reaction mixture was diluted with saturated ammonium chloride solution and extracted three times with 50 ml of ether. The combined organic portions were washed twice with water and once with brine and dried with anhydrous magnesium sulfate. The residue upon removal of solvent was dissolved in 5 ml of methylene chloride and placed onto a column of acid-washed silica gel (25 g of silica gel 60, E. Merck cat. no. 7736, previously washed with 6 N hydrochloric acid, water to neutrality, saturated disodium EDTA solution, water, and then 0.3 N hydrochloric acid and dried in air for 72 hours, was slurry-packed in hexane). Elution was carried out under a positive pressure of nitrogen with hexane (100 ml), ether (100 ml), 10:1 ether:acetonitrile (150 ml), and 100:10:2 ether:acetonitrile:triethylamine. The last solvent mixture brought about elution of α-vinylornithine methyl ester.

Example 2

Following the procedure of Example 1B), *o*-methoxybenzyl bromide is used in place of the N-benzylidine-3-amino-1-bromo-propane, to prepare methoxy-α-vinyl-*o*-tyrosine methyl ester, which is used as intermediate in Example 6.

Example 3

Following the procedure of Example 1B), 2,5-dimethoxybenzyl bromide is used in place of the N-benzylidine-3-amino-1-bromo-propane to prepare 2,5-dimethoxybenzyl-α-vinylglycine methyl ester, which is used as intermediate in Example 7.

Example 4

Following the procedure of Example 1B), gramine methiodide is used in place of the N-benzylidine-3-amino-1-bromo-propane to prepare α-vinyl-tryptophan methyl ester.

Example 5

A mixture of 1.301 g of α-N-tosylimidazoylmethyl-α-vinylglycine methyl ester and 15 ml of concentrated hydrochloric acid was heated at reflux for 5 hours. The reaction mixture was cooled, extracted three times with 5 ml of methylene chloride, and treated with activated charcoal. Filtration and evaporation of solvent, and then purification by ion-exchange chromatography[(1)] gave 0.695 g of α-vinylhistidine dihydrochloride as an amorphous solid. NMR ($D_2O$) showed: 3.42 (2H, s), 5.32 (1H, d, J=16), 5.48 (1H, d, J=10), 6.05 (1H, d of d, J=16, 10), 7.28 (1H, d, J=1), 8.52 (1H, d, J=1). TLC showed a single spot, $R_f$=0.3 (silica gel, 50:8:20 butanol:acetic acid:water).

Example 6

Using the Example 5 procedure, methoxy-α-vinyl-*o*-tyrosine methyl ester is treated to give *o*-methoxy-α-vinylphenylalanine · HCl.

---

[(1)]on Dowex 50W-X4 (Trade Mark) cation exchange resin.

Example 7

Using the Example 5 procedure, 2,5-dimethoxybenzyl-$\alpha$-vinylglycine methyl ester is treated to give 2,5-dimethoxy-$\alpha$-vinylphenylalanine hydrochloride.

Example 8

Using the procedure of Example 1B, alkylation was carried out with N-benzylidine-3-amino-1-bromo-propane. After workup with aqueous ammonium chloride as described, the crude product was mixed with 100 ml of 6 N hydrochloric acid and the well-stirred mixture was heated at 110°C for 2 hours. The solution was cooled, extracted four times with 10 ml of methylene chloride, and treated with activated charcoal. The solution was filtered and evaporated, and the residue purified on Dowex 50W-X4 (Trade Mark) cation exchange resin, eluted first with water and then with 2 N hydrochloric acid, giving 0.560 g of $\alpha$-vinylornithine dihydrochloride as a white foam. NMR ($D_2O$) showed: 1.4—2.2 $\delta$ (4H, m), 2.8—3.1 (2H, m), 5.15 (1H, d, J=18), 5.25 (1H, d, J=10), 5.87 (1H, d of d, J=18, 10).

Example 9

$\alpha$-Vinyltryptophan

Treatment of $\alpha$-vinyltryptophan methyl ester in 1:1 methanol:water with two equivalents of sodium hydroxide, neutralization of the reaction mixture with 1 N aqueous hydrochloric acid, and purification of the crude amino acid on Dowex 50W-X4 (Trade Mark) cation exchange resin (elution with water and then 2 N aqueous hydrochloric acid) gives $\alpha$-vinyltryptophan hydrochloride.

Example 10

The procedure described in Example 1B is carried out except that 5-methoxymethylgramine methiodide (5 mmol) is used in place of N-benzylidine-3-amino-1-bromo-propane and that the crude product is not submitted to the silica gel treatment but is instead dissolved in 1:1 methanol:water (50 ml) and treated with two equivalents of potassium hydroxide. After five hours, the reaction mixture is acidified to pH=3 by addition of 1 N hydrochloric acid and the resulting solution is refluxed for 5 minutes. The reaction mixture is cooled, just neutralized with aqueous potassium bicarbonate, and concentrated to dryness under vacuum. Purification of the crude amino acid on Dowex 50W-X4 (Trade Mark) cation exchange resin (elution with water and then 2 N aqueous hydrochloric acid) gives 5-hydroxy-$\alpha$-vinyltryptophan hydrochloride.

The products obtained in the examples are racemates. These racemates may be resolved using conventional techniques, e.g., by treating the racemate with a suitable optically active acid in a suitable solvent and recovering the individual amino acid isomer from the resultant salt.

**Claims for the Contracting States: BE, CH, DE, FR, GB, IT, LU, NL, SE**

1. Compounds having the formula

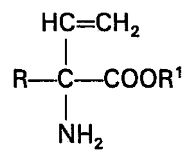

and pharmaceutically acceptable salts thereof wherein R is

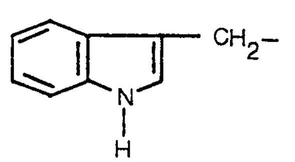 , 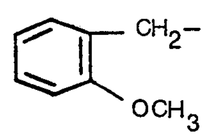 , 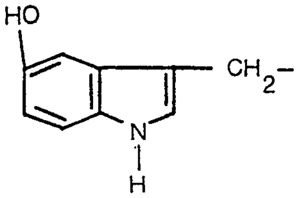 ,

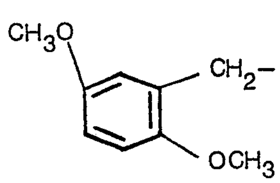 , 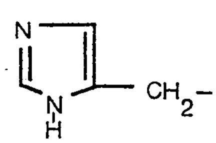 ,

or

$H_2N—(CH_2)_2—CH_2—$

and $R^1$ is H or $C_1—C_{18}$alkyl.

2. Compounds of Claim 1, wherein $R^1$ is $C_1$—$C_6$alkyl.
3. Compounds of Claim 1, wherein $R^1$ is H.
4. Compounds of Claim 1 having the L-isomer configuration.
5. Compounds of Claim 1, wherein R is

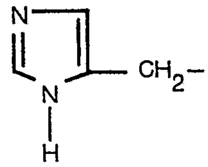

6. Compounds of Claim 1 having the formula:

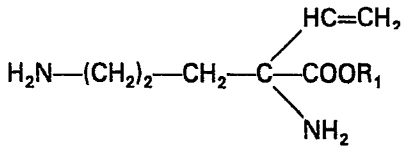

7. Compounds of Claim 1, wherein R is

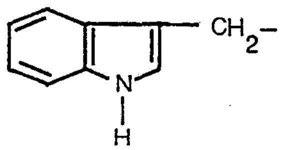

or

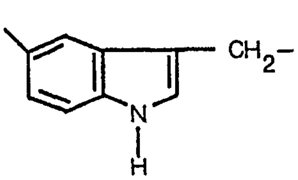

8. Compounds of Claims 5, 6 or 7, wherein (a) $R^1$ is H or (b) $R^1$ is H and said compound is the L-isomer.

9. A pharmaceutical composition containing a therapeutically effective amount of a compound of Claim 1.

**Claims for the contracting state: AT**

1. A process for preparing compounds of the formula

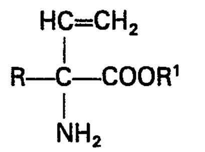

I

and pharmaceutically acceptable salts thereof where R is

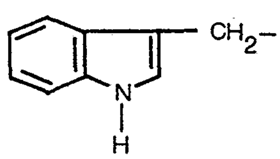

,

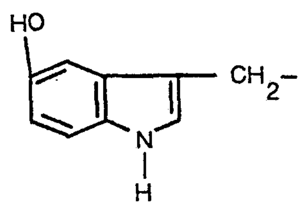

,

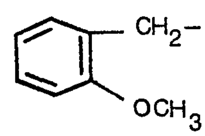

,

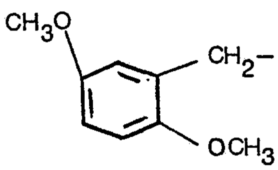

,

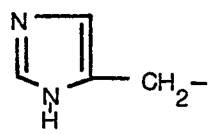

or

$H_2$—N—$(CH_2)_2$—$CH_2$—

and $R^1$ is H or $C_1$—$C_{18}$alkyl which comprises

1) reducing a compound of the formula

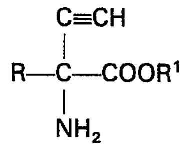

where $R^1$ is H or $C_1$—$C_{18}$alkyl or

2) for preparing compounds of the formula I wherein $R^1$ is H, hydrolyzing a compound of the formula

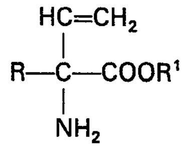

where $R^1$ is $C_1$—$C_{18}$alkyl, and optionally converting the obtained compound into a pharmaceutically acceptable salt thereof.

2. The process of Claim 1, wherein R is

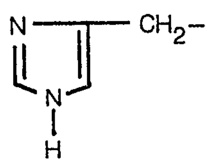
,
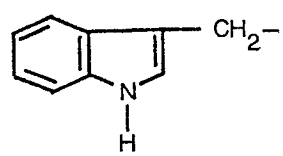
,
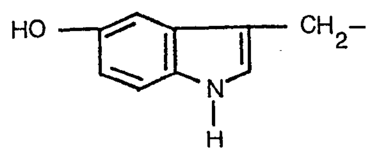

or

$H_2N$—$(CH_2)_2$—$CH_2$—.

**Revendications pour les Etats Contractants: BE, CH, DE, FR, GB, IT, LU, NL, SE**

1. Composés de formule

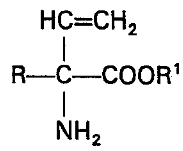

et leurs sels pharmaceutiquement acceptables, où R représente

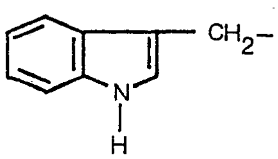
,
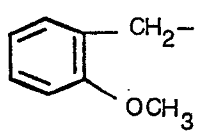
,
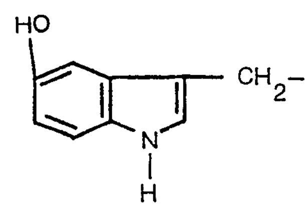
,

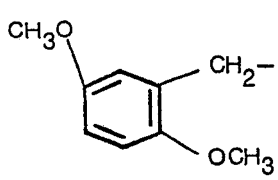
,
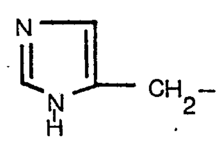
,

ou

$H_2N$—$(CH_2)_2$—$CH_2$—

et $R^1$ représente H ou un alcoyle en $C_1$ à $C_{18}$.

2. Composés de la revendication 1 où $R^1$ est un alcoyle en $C_1$ à $C_6$.

3. Composés de la revendication 1 où $R^1$ représente H.

4. Composés de la revendication 1 ayant la configuration isomérique L.

5. Composés de la revendication 1 où R représente

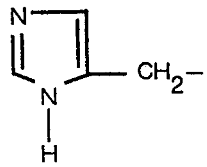

6. Composés de la revendication 1 de formule

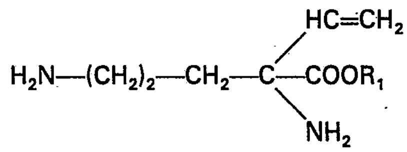

7. Composés de la revendication 1 où R représente

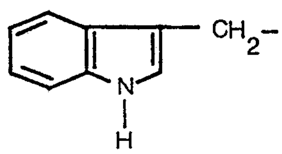

ou

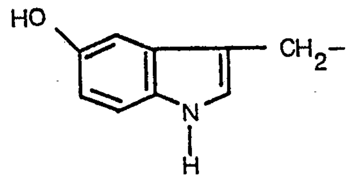

8. Composés des revendications 5, 6 ou 7 où (a) $R^1$ représente H ou (b) $R^1$ représente H et ledit composé est l'isomère L.

9. Composition pharmaceutique contenant une quantité thérapeutiquement efficace d'un composé de la revendication 1.

**Revendications pour l'etat contractant: AT**

1. Procédé de préparation de composés de formule

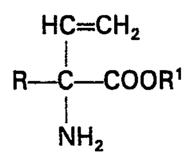

I

et de leurs sels pharmaceutiquement acceptables où R représente

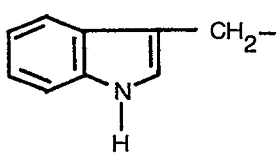

,

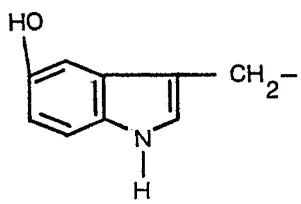

,

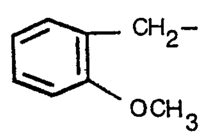

,

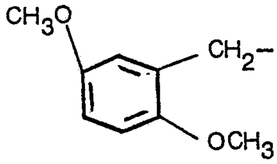

,

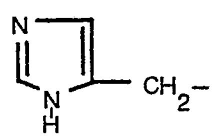

ou

$H_2—N—(CH_2)_2—CH_2—$

et $R^1$ représente H ou un alcoyle en $C_1$ à $C_{18}$ dans lequel

1) on réduit un composé de formule

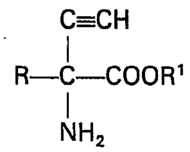

où $R^1$ représente H ou un alcoyle en $C_1$ à $C_{18}$ ou

2) pour préparer des composés de formule I où $R^1$ représente H, on hydrolyse un composé de formule

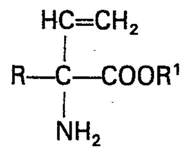

où $R^1$ est un alcoyle en $C_1$ à $C_{18}$, et on transforme éventuellement le composé obtenu en un sel pharmaceutiquement acceptable de ce composé.

2. Procédé de la revendication 1 où R représente

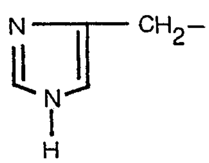 , 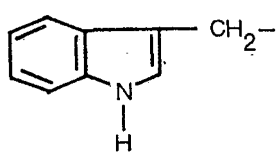 , 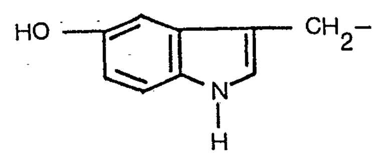

ou

$H_2N—(CH_2)_2—CH_2—$.

**Patentansprüche für die Vertragsstaaten: BE, CH, DE, FR, GB, IT, LU, NL, SE**

1. Verbindungen der Formel

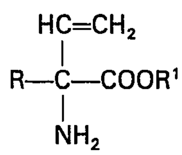

und pharmazeutisch annehmbare Salze davon, worin R

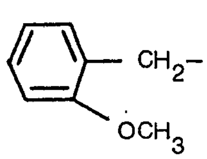 , 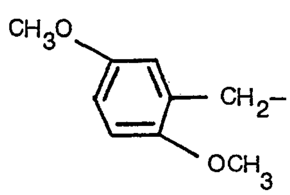 , 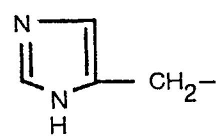 ,

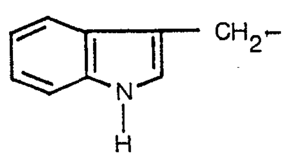 , 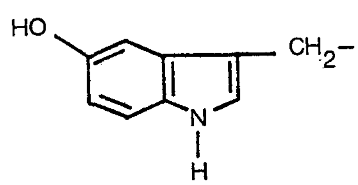

oder

$H_2N—(CH_2)_2—CH_2—$

ist und $R^1$ H oder $C_1—C_{18}$-Alkyl ist.

2. Verbindungennach Anspruch 1, worin $R^1$ $C_1—C_6$-Alkyl ist.

3. Verbindungen nach Anspruch 1, worin $R^1$ H ist.

4. Verbindungen nach Anspruch 1, welche die L-Isomerkonfiguration aufweisen.

5. Verbindungen nach Anspruch 1, worin R

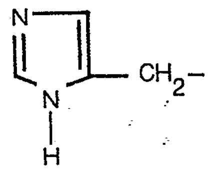

ist.

6. Verbindungen nach Anspruch 1 mit der Formel:

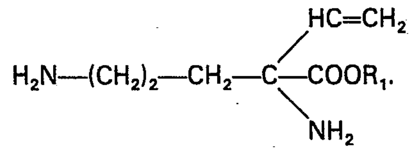

7. Verbindungen nach Anspruch 1, worin R

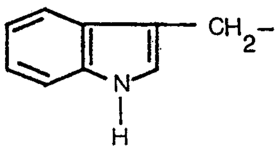 oder 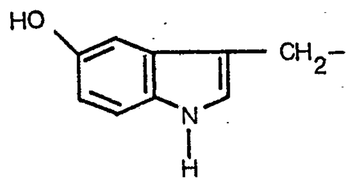

ist.

8. Verbindungen nach Anspruch 5, 6 oder 7, worin (a) $R^1$ H ist oder (b) $R^1$ H ist und diese Verbindung das L-Isomer ist.

9. Eine pharmazeutische Zusammensetzung, enthaltend eine therapeutisch wirksame Menge einer Verbindung nach Anspruch 1.

**Patentansprüche für den Vertragsstaat: AT**

1. Ein Verfahren zur Herstellung von Verbindungen der Formel

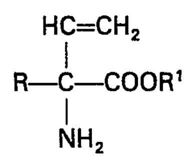

und pharmazeutisch annehmbaren Salzen davon, worin R

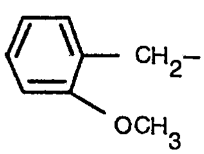 , 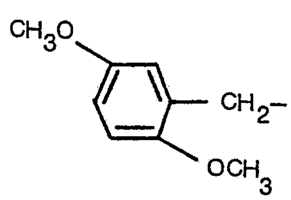 , 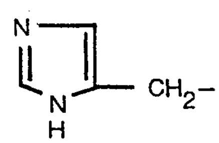 ,

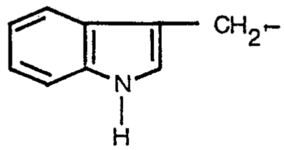 , 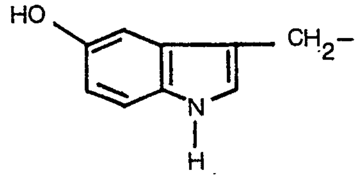

oder

$H_2N—(CH_2)_2—CH_2—$

ist und $R^1$ H oder $C_1$—$C_{18}$-Alkyl ist, umfassend

1) die Reduktion einer Verbindung der Formel

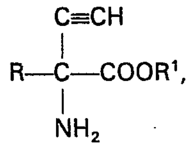

worin $R^1$ H oder $C_1$—$C_{18}$-Alkyl ist, oder

2) zur Herstellung von Verbindungen der Formel I, worin $R^1$ H ist, das Hydrolysieren einer Verbindung der Formel

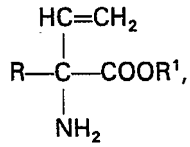

worin $R^1$ $C_1$—$C_{18}$-Alkyl ist, und gegebenenfalls das Umwandeln der erhaltenen Verbindung in ein pharmazeutisch annehmbares Salz davon.

2. Das Verfahren nach Anspruch 1, worin R

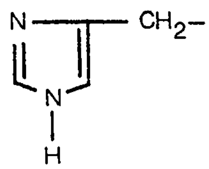 , 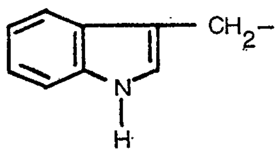 , 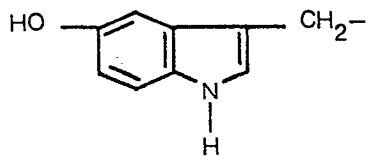

oder

$$H_2N—(CH_2)_2—CH_2—$$

ist.